# EUROPEAN PATENT SPECIFICATION

(11) **EP 3 226 926 B1**
(45) Date of publication and mention of the grant of the patent: **05.08.2020**
(21) Application number: 15823016.9
(22) Date of filing: 02.12.2015
(51) Int. Cl.: A61L 31/16, A61K 9/00

(54) **BIODEGRADABLE POLYMER MEDICAL DEVICE**
MEDIZINPRODUKT AUS BIOLOGISCH ABBAUBAREM POLYMER
DISPOSITIF MÉDICAL EN POLYMÈRE BIODÉGRADABLE

(30) Priority: 03.12.2014 IT BS20140201
(43) Date of publication of application: 11.10.2017
(73) Proprietor: Testa, Gianluca, 86100 Campobasso (IT); Ambrosone, Luigi, 86100 Campobasso (IT); Costagliola, Ciro, 86100 Campobasso (IT)
(72) Inventor: Testa, Gianluca, 86100 Campobasso (IT); Ambrosone, Luigi, 86100 Campobasso (IT); Costagliola, Ciro, 86100 Campobasso (IT)
(74) Representative: Sangiacomo, Ines
(86) International application number: PCT/IB2015/059278
(87) International publication number: WO 2016/088054

(56) References cited:
- EP-A2- 0 160 266
- US-A1- 2007 148 251
- US-A1- 2008 051 882
- US-A1- 2008 234 657
- US-A1- 2009 047 318
- Chuanglong He ET AL: "Crosslinking of poly(L-lactide) nanofibers with triallyl isocyanutrate by gamma-irradiation for tissue engineering application", JOURNAL OF BIOMEDICAL MATERIALS RESEARCH. PART A, vol. 99A, no. 4, 27 September 2011 (2011-09-27), pages 655-665, XP055483095, HOBOKEN, NY, US ISSN: 1549-3296, DOI: 10.1002/jbm.a.33235

## Description

### Field of the invention

In its most general aspect the present invention relates to the medical and veterinary field and in particular it concerns a device, hereinafter only referred to as medical device to be used in both the aforementioned areas, for example but not limited to a stent, for the controlled release of at least one active substance, for example but not limited to a drug, in a subject, for example but not limited to a mammal such as a human being.

More particularly, the present invention relates to a device of the aforementioned type, made of biodegradable polymeric material, and to a related method of realization.

### State of the art

It is well known that a stent is a cylindrical structure, usually metallic, meshed, which is introduced into a hollow organ lumen of a patient, for example in the viscera, or in a blood vessel, and is expanded until its diameter is equal to that of the lumen.

A stent of this type has a typically structural function consisting of mechanical support and is used for example to reduce stenosis, exclude an aneurysm or, however, to maintain patent the organ or the vessel in which it is implanted.

There are also metal stent of the aforementioned type loaded with drugs for its local release, which, however, has always, primarily, a structural function.

Finally, there are reabsorbable stent in biodegradable material, consisting of a biopolymer and a drug, which have a time limited structural function and which are used always for a local release of the drug.

In a stent of the latter type, a polymer matrix, for example polylactic acid (PLA), incorporates the drug which is released locally, and likely also gradually, inside the patient, while the polymer matrix is metabolized by the organism of the patient and in particular degraded into elementary compounds such as, in the case of PLA, carbon dioxide and water.

Although beneficial, stents of the aforementioned types are not free from drawbacks.

Metal stent, both those loaded with drugs and those without drugs, for example, are permanent, and then remain permanently at the implant site (in situ) once implanted.

Moreover, the possible loading with a drug is particularly difficult and the chemical-physical characteristics of the drugs suitable for use with such stents cannot guarantee the local pharmacological effects intended and expected.

In the biodegradable stent provided in the prior art, however, the release of a desired drug, although theoretically independent, is in fact influenced by the degradation of the polymeric matrix.

In fact, although the polymer matrix is theoretically conceived to completely degrade in a time interval longer than that programmed or hypothesized for the total release of the drug, the slow but constant erosion of the polymer in the lumen of the patient determined by its degradation makes the structure of the stent progressively more fragile, hence unstable, resulting in the possible uncontrolled release of the drug, which can possibly be programmed with adequate precision only as regards the first period of use of the stent.

In these cases, therefore, in line with the primarily structural function that also the third-generation stents have, a long-term therapy based on a controlled and prolonged release of a drug is not effective, since the duration of the polymer does not provide reliable appraisal of drug's fate, and in particular on the drug's release at the implant site.

In addition, all the above mentioned drug releasing stents, always in line with their fundamentally structural function, allow only a local drug release limited to the wall of the lumen in contact with the stent (intramural release), hence a radial release outward of the stent.

The above is in agreement with the fact that the stent according to the prior art are used in the treatment of stenoses in the lumen of the organ in which they are implanted.

To date, therefore, the prior art does not provide medical devices of the type of a stent, or other devices intended to be implanted in a vessel or in a hollow organ of a patient, able to satisfy multiple needs, including effective drug long term therapies such as, for example but not only, therapies for the treatment or cure of pulmonary hypertension.

Pulmonary hypertension (PH) is a hemodynamic and pathophysiological state defined as the increase of mean pulmonary artery pressure (mPAP) ≥25 mmHg at rest as assessed by right heart catheterization (RHC).

Pulmonary hypertension can be classified as pre-capillary pulmonary hypertension, when due to idiopathic pulmonary vascular disease, toxic drugs, lung disease, thromboembolic disease, congenital heart diseases and other clinical conditions, or can be classified as post-capillary pulmonary hypertension when is due to left heart disease.

Pre-capillary pulmonary hypertension is a progressive condition that affects either distal or proximal pulmonary arteries and is characterized by pathological lesions involving all the three layers of the pulmonary vessels (intima, media and adventitia) while pulmonary veins are classically unaffected.

These lesions are, depending on the underlying disease, medial hypertrophy, intimal proliferative and fibrotic concentric and eccentric changes, intimal obstructive proliferation, adventitial thickening with moderate perivascular inflammatory infiltrates, plexiform and dilated lesions and thrombotic lesions or organized thrombi tightly attached to the pulmonary arterial medial layer in the elastic pulmonary arteries that may completely occlude the lumen or form different grades of stenosis, webs, and bands.

In post-capillary pulmonary hypertension pathological lesions are characterized by enlarged and thickened pulmonary veins, pulmonary capillary dilatation, interstitial edema, alveolar hemorrhage, and lymphatic vessel and lymph node enlargement and distal pulmonary arteries may be affected by medial hypertrophy and intimal fibrosis.

In pre-capillary pulmonary hypertension, the primary event leading to the rise of pulmonary arterial pressure is the increase of pulmonary vascular resistance due to intimal and medial proliferation.

This progressive condition ultimately results in right heart failure and steep clinical worsening which significantly contributes to the poor prognosis observed in patients with pre-capillary pulmonary hypertension.

The most important biochemical features involved in the development of pulmonary arteries dysfunction in pre-capillary pulmonary hypertension are the hyper-activation of the endothelin-1 pathway, a reduction of nitric oxide (NO) production and release and a the reduction of the levels of prostacyclin (PGI2).

These pathways are currently targeted by approved drugs for pre-capillary pulmonary hypertension.

Percutaneous transluminal angioplasty (PTA) is a procedure, first introduced in clinical practice in the '70s, that nowadays represents a valuable option in patients requiring coronary artery (PTCA) or peripheral artery revascularization.

Since the first introduction in the clinical practice of angioplasty, technology has provided the operators with several instruments to improve results and clinical outcomes. Among these the implant of the stent in the stenotic vessel has the aim to prevent re-occlusion.

In particular, research has greatly improved the materials of which stents are composed, trying to effectively cojugate the mechanical function of maintaining a stenotic vessel dilated and the therapeutic function of local drug delivery to reduce the rate of restenosis.

Over the years, there have been introduced in clinical practice intravascular devices which are able to both release drugs and being reabsorbed over time, so that vascular physiology is not permanently altered.

In particular the use of fully bioabsorbable polymers has led to the production of stents made of polylactic acid (PLA) which locally release drugs.

In detail, PLA is metabolized through the Krebs cycle to be completely degraded over a defined timeframe that can reach the two years.

Over the last decades the use of specific pharmacological agents in the treatment of selected patients affected by pre-capillary pulmonary hypertension has led to substantial improvement of clinical status and prognosis.

Currently the most used drugs specifically inhibit the activity of the endothelin-1 pathway (Endothelin Receptor Antagonists, ERA), increase the levels of NO via inhibition of the phosphodiesterase-5 pathway (Phosphodiesterase-5 inhibitors, PDE5i) and increase the activity of prostacyclin via direct administration of prostacyclin analogues (prostanoids).

These drugs are administered orally (ERA, PDE5i), continuously infused intravenously (IV) or subcutaneously (SC) (prostanoids) or inhaled (prostanoids).

Despite the significant increase of the use of these specific drugs morbidity and mortality of patients affected by pre-capillary pulmonary hypertension still remain high.

Epoprostenol, a synthetic prostacyclin analogue, was the first drug approved by FDA for the treatment of pre-capillary pulmonary hypertension in 1995.

Prostacyclin analogues are very effective in the treatment of pre-capillary pulmonary hypertension but have a very short circulating half-life ranging from ≈6 minutes to 4 hours, depending on the drug and on the administration route (IV or SC) and are recommended for the intravenous use in patient with high degree of functional decline which are refractory to oral or inhaled drugs.

The use of intravenous or subcutaneous prostanoids carries several practical limitations related to its administration route.

Indeed the continuous intravenous or subcutaneous administration, currently recommended, by means of a permanent indwelling catheter represents a significant clinical challenge for both patients and care providers.

In fact, dosing interruptions and IV or SC catheter infections, which are undesirable side events of this administration route, can be life-threatening.

Because of these practical limitations only experienced centers should provide this therapy thus making IV and SC prostanoids substantially underused in current practice.

Only recently it has been hypothesized that the first-line approach with a combined therapy consisting of ERAs, PDE5i and intravenous or subcutaneous prostanoids might be more effective than the established sequential approach in which drugs are added on as clinical and/or functional status worsens.

This idea is still poorly supported by evidences and is not recommended, yet very attractive.

Moreover, the first line use of prostanoids would expose patients with early stage disease to the challenge of managing the continuous intravenous administration and all the related risks.

Nevertheless it has been recently demonstrated that in patients with pre-capillary pulmonary hypertension the first-line use of intravenous prostanoids alone or in combination with oral drugs leads to a significant improvement of symptoms and survival.

US 2007/148251 A1 discloses an implantable medical device having a coating comprising nanoparticles, in which the nanoparticles comprise a bioactive agent.

EP 0 160 266 A2 discloses a liposome composition comprising a crosslinked material having a three-dimensional network structure, and a liposome physically entrapped within the network structure and enclosing a material to be administered into a living body.

US 2009/0047318 A1 discloses an implantable nanoparticle-coated medical devices, in which the nanoparticles comprise one or more active agents.

US 2008/234657 A1 discloses a method using a biodegradable polymer stent for administering at least a bioactive material to the first proximal third of coronary arteries, in which the bioactive material can be included in the core of nanoparticles.

US 2008/0051882 A1 discloses an intra-lumenal drug delivery devices that is coated or impregnated with a pharmaceutical agent suitable for use in treatment of restenosis, pulmonary hypertension, and cancer.

### Summary of the Invention

The technical problem underlying the present invention was to provide a medical device having structural and functional characteristics such as to overcome one or more of the abovementioned drawbacks, and in particular a medical device made of biodegradable polymeric material, conceived to be implanted in a lumen of a subject, apt to the programmed, controlled and preferably not locally limited release of a given drug associated with the polymeric material, and hence a medical device apt to release the drug for the entire period of the life of the medical device, so for the whole period in which it is usefully implanted in the lumen of the subject, regardless of the erosion of the polymeric material, preferably at least towards the interior of the lumen in which it is implanted, and hence a medical device of the above type apt to be used for a long-term drug therapy, essentially having a functional role of vector of the drug.

In accordance with the invention, the abovementioned problem is solved by a medical device encompassing a body or hollow element with an essentially circular section substantially made of a biodegradable polymeric matrix and at least one active substance associated with the abovementioned hollow body, which is characterized by the fact that it comprises at least one support for containment and release of the abovementioned at least one active substance, wherein said at least one support comprises a plurality of nanometric sized elements consisting of liposomes, wherein the abovementioned at least one support is dispersed in and/or on the abovementioned biodegradable polymer matrix, in which said biodegradable polymer matrix is a polymer or a copolymer of a polylactic acid (PLA/ PLLA) at least partially crosslinked and wherein said at least one support is entrapped in said polymer matrix.

Indeed, in the device according to the invention, the aforementioned at least one active substance and the abovementioned biodegradable polymer matrix are, so to say, built-in but among themselves physically separate, the said at least one active substance being encapsulated in, so to speak, and released from, the said at least one support, which usefully allows to program and control the release of the at least one active substance regardless of the degradation of the biodegradable polymer matrix, the erosion of the biodegradable polymer matrix representing the slow stage of the process of release of the at least one active substance.

Preferably, the said at least one support is dispersed on and/or in the biodegradable polymer matrix at least at an inner surface or an inner portion of the said hollow body with the essentially circular section, in which by inner portion it is intended a portion radially extended from the said inner surface up to before an outer surface of said hollow body opposite to said inner surface.

Preferably the said polymer matrix is entirely crosslinked.

The above-mentioned biodegradable polymer matrix comprises a polymer or a copolymer, of a polylactic acid (PLA/PLLA), e.g. a poly (lactic-co-glycolic) acid (PLG).

Preferably the abovementioned copolymer is a block copolymer, more preferably a block copolymer, cross-linked with a mesh pattern size equal to or less than about 10 nm, more preferably with a mesh size comprised between 6 and 10 nm, even more preferably with continuous blocks domains of the same monomeric unit of length comprised between 8 and 15 nanometers.

Preferably the abovementioned at least one active substance is selected from the group comprising: cholinergic receptor stimulating agents and cholinesterase inhibitors, cholinergic receptor blocking agents, sympathomimetic agents, adrenergic receptor blockers, antihypertensive substances, venous and/or arterial vasodilator substances, substances active in the treatment of angina pectoris, substances active in the treatment of cardiac arrhythmias, diuretics, histaminic substances, serotonin, vasoactive 5 peptides, eicosanoids including prostaglandins, thromboxanes, leukotrienes and related substances for treatment of obstructive pulmonary disease, sedatives/hypnotic agents, anticonvulsant substances, substances for the treatment of Parkinson's disease, 10 lithium based antidepressant and/or antipsychotic substances, local or general anesthetic and/or analgesic substances, substances for smooth muscle relaxation, hematopoietic growth factors, substances for the treatment of coagulation disorders, substances used for the treatment 15 of dyslipidaemia, NSAIDs, anti-rheumatic substances, non-opioid analgesics, substances for the treatment of bones and for the treatment of mineral homeostasis, hormones, antithyroideal antagonistic substances, antidiabetics, antibiotic substances, chemotherapeutic agents, antifungal 20 agents, antifungal microbial and antiviral substances, biological drugs, nutraceuticals, pesticides, chelating agents or mixtures thereof.

Preferably the abovementioned at least one active substance is a substance for the treatment or diagnosis of a patient, more preferably for the care and treatment of pulmonary hypertension, and even more preferably is epoprostenol.

Preferably the abovementioned support comprises liposomes obtained from phosphatidylcholine (Epikuron 200, lecithin) mixed in water.

Preferably the aforesaid nanometric elements are covalently bound to said biodegradable polymeric matrix cross-linked at points or nodes of crosslinking of the biodegradable polymer matrix, the abovementioned nanometric elements constituting the same nodes of crosslinking of the biodegradable polymer matrix, not excluding however the possibility of providing nanometric elements, also or only, covalently bonded to, or between, the monomeric units of the biodegradable polymer matrix in different points from the nodes of crosslinking.

Preferably the aforesaid nanometric elements covalently bound to the abovementioned biodegradable polymeric matrix are functionalized, for example coated with molecules of polyethylene glycol having terminal amino groups.

Preferably, the above-mentioned biodegradable polymer matrix is functionalized with chemical groups appropriate to be used as docking sites for the immobilization of the aforesaid nanometric elements.

In accordance with the invention the above problem is also solved by a process of production of a medical device of the abovementioned type, essentially comprising the steps of:
- a) providing at least one support of the aforementioned type;
- b) load the abovementioned at least one support with at least one prefixed active substance of the abovementioned type;
- c) providing a biodegradable polymer matrix of the abovementioned type;
- d) immobilizing the abovementioned at least one support loaded with the abovementioned at least one active substance on and/or in the abovementioned biodegradable polymer matrix;
- e) shaping the above-mentioned biodegradable polymer matrix in the form of a hollow body having a substantially circular section;
- f) cross-link the abovementioned biodegradable polymer matrix, wherein at least part of said step d), wherein at least one support is immobilized within and/or on said biodegradable polymer matrix, coincides with said step f), wherein said polymer matrix is crosslinked.

In accordance with the invention the abovementioned step d), wherein the at least one support is immobilized in and/or on the aforementioned biodegradable polymer matrix, may be conducted prior or subsequent to the aforementioned step e), wherein the abovementioned biodegradable polymer matrix is shaped in the form of body or hollow element with essentially circular section.

Basically, according to the above and according to the present invention, it is provided a medical device in which the release of the at least one active substance takes place primarily with the at least one support still associated with the biodegradable polymer matrix, the biodegradable polymeric matrix degradation taking place substantially independently of the release of the at least one active substance, preferably taking place once the release is essentially completed.

The kinetics of the release process of the at least one active substance from the at least one support is different from the kinetics of the process of degradation of the biodegradable polymeric matrix, the person skilled in the art being able to pre-calculate and calibrate the relative equations in order to optimize the release timing of the at least one active substance.

Generally speaking, it can be said that the kinetics of the above two processes are dictated, in addition to the chemical affinity between the different compounds involved, also by static and dynamic conditions of the fluids flowing in the lumen of the subject involved, hence into the lumen of the medical device.

In particular, the process of release of the at least one active substance, and then its exit from the above nanometric elements, as well as possibly the degradation process of the biodegradable polymeric matrix, for example the erosion, can be favored by the so-called phenomenon of the "swelling ".

For example, hydrophilic compounds spreading into the biodegradable polymeric matrix, between the meshes of the lattice of the biodegradable polymeric matrix, and breaking into at the outer portions of nanometric elements, such as shell portions, can determine bulges such as to trigger any breakages of the nanomeric elements, resulting in the acceleration of the process of release of the at least one active substance.

The above-mentioned phenomenon of "swelling" does not seem quite manageable, but in fact the causes that generate it are quite well known.

Therefore, in the use of this medical device, the person skilled in the art will minimize the side effects of "swelling".

Still, it has to be added that the abovementioned nanometric elements, i.e. liposomes, are not rigidly static entities, but are more like a sort of containers dynamically pulsatile in the environment in which they are introduced.

Indeed, there is a thermodynamic equilibrium between a so-called "open" form and a so-called "closed" form which can be modified and moved towards the one or the other form, according to the needs, in accordance with the knowledge of the skilled person in the art.

In this regard it has to be said that the at least one active substance and the abovementioned nanometric elements are held together by weak forces such as van der Waals bonds, hydrogen bonda, London dispersion, which can be easily overcome.

From a kinetic point of view, this implies that the process of breaking and/or opening of the nanometric elements takes place more rapidly as compared to what would occur if strongest interactions were involved, such as for example the same number of covalent bonds, which, instead, hold together the monomeric units of the biodegradable polymer matrix, and also the monomeric units chains in the case of biodegradable crosslinked polymeric matrix, and which again, in accordance with a preferred embodiment of the invention, hold together the elements of nanometric dimensions to the biodegradable polymeric matrix, optionally crosslinked, for example at the level of the nodes of crosslinking.

Therefore, the fast stage between the two abovementioned processes of release of the at least one active substance and of degradation of the biodegradable polymer matrix is represented by the process of release of the at least one active substance, while the slow stage is represented by the degradation, namely breakage, of the biodegradable polymer matrix.

In case the elements of nanometric dimensions constitute the aforesaid nodes of crosslinking, the higher the nodal density of the biodegradable polymeric matrix, the higher will be the dosage of the at least one active substance and the more this will be evenly distributed within and on the surface of medical device. Rather it will be the density of the nodes to modulate the release rate of the active principle.

### Definitions

In the context of the present invention the terms subject and patient are used interchangeably and include mammals, such as for example a human, and animals in general, including non-mammals, and therefore indicate any subject in which the present medical device can be implanted.

In the context of the present invention the expressions drug and active substance are interchangeable, and include substances for the cure, treatment, or prophylaxis of a disease or a disorder in a subject, or for the diagnosis of a disease or a disorder in a subject, such as a contrast medium or a marker.

In the context of the present invention the term body or hollow element essentially circular cross-section indicates a ring, a truncated hollow cone, or a hollow cylinder of reduced dimensions, for example but not limited to a stent, to be implanted in a lumen of a patient.

In the context of the present invention the expression lumen is to be intended in its common meaning used in the medical or veterinary use, and therefore refers to any passage in an organ or in a hollow body of a patient.

In the context of the present invention the expression biodegradable polymer matrix is to be understood in its common meaning used in the field of polymers, and in particular means a polymeric material, with a basis of one or more monomers, thus also a copolymer, optionally crosslinked, which naturally decomposes into simple or elementary products.

### Detailed Description of the figure

Further characteristics and advantages of the invention will become more apparent from an examination of the following detailed description of some preferred but not exclusive embodiment, illustrated only by way of non-limiting example, with the support of the attached drawing in which:

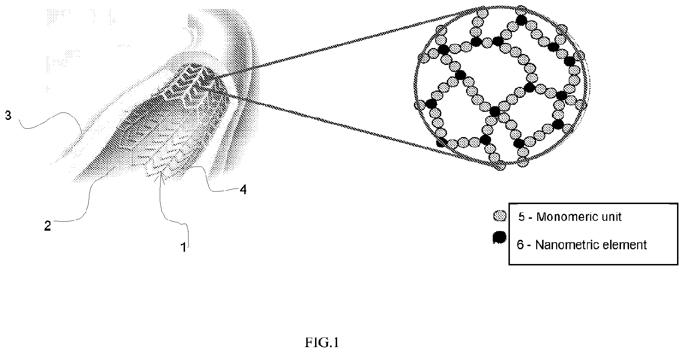

- Figure 1 schematically illustrates a medical device in accordance with an embodiment of the present invention, with a corresponding enlarged detail, implanted in a lumen of a blood vessel of a subject, for example a human being.

### Detailed Description of the invention

The present invention relates to a medical device apt to be implanted in a lumen of a patient, for the controlled release of a drug, or one or more active substances, in particular for the care, treatment or prophylaxis of a pathology or a disorder in a subject, or for the diagnosis of a disease or a disorder in a subject.

The medical device according to the invention is defined in claim 1, and essentially comprises a body or a hollow member with a substantially circular section made of a biodegradable polymeric matrix to which is associated the drug, and a support of containment and release of the drug dispersed on and/or in the biodegradable polymer matrix.

In accordance with one aspect of the invention, the support of containment and release of the drug is a support of nanometric size, and comprises a plurality of nanometric elements consisting of liposomes.

The elements of nanometric dimensions in practice represent a sort of nanocapsules containing the drug, which are incorporated in, and/or supported by, biodegradable polymer matrix, and therefore are dispersed in and/or on the biodegradable polymer matrix, which are able to release the drug at a speed advantageously programmable and controlled.

With regard to the biodegradable polymer matrix it has to be added that it is a polymer or a copolymer of a polylactic acid (PLA/PLLA), e.g. a poly (lactic-co-glycolic) acid (PLG).

The polymer matrix is at least partially, or entirely, crosslinked.

In the case of copolymer, the polymer matrix of biodegradable medical device according to the invention has a structure so to say ordered, the copolymer preferably being a block, with constant domains of blocks of the same monomer unit having a length between about 8 nm and about 15 nm.

Still, in the case of cross-linking, the polymer matrix of biodegradable medical device according to the invention has a structure with a mesh pattern size equal to or less than about 10 nm, more preferably a mesh size lying between 6 and 10 nm, and then with mesh that the limit is comparable to the pores.

Still in accordance with the invention, the support of nanometric dimensions is physically entrapped in the polymer matrix, preferably at least at an inner surface or an inner portion of the hollow body.

With regard to the active substance, it has to be said that it is chosen from the group comprising: cholinergic receptor stimulating agents and cholinesterase inhibitors, cholinergic receptor blocking agents, sympathomimetic agents, adrenergic receptor blockers, antihypertensive substances, venous and/or arterial vasodilator substances, substances active in the treatment of angina pectoris, substances active in the treatment of cardiac arrhythmias, diuretics, histaminic substances, serotonin, vasoactive 5 peptides, eicosanoids including prostaglandins, thromboxanes, leukotrienes and related substances for treatment of obstructive pulmonary disease, sedatives/hypnotic agents, anticonvulsant substances, substances for the treatment of Parkinson's disease, 10 lithium based antidepressant and/or antipsychotic substances, local or general anesthetic and/or analgesic substances, substances for smooth muscle relaxation, hematopoietic growth factors, substances for the treatment of coagulation disorders, substances used for the treatment 15 of dyslipidaemia, NSAIDs, anti-rheumatic substances, non-opioid analgesics, substances for the treatment of bones and for the treatment of mineral homeostasis, hormones, antithyroideal antagonistic substances, antidiabetics, antibiotic substances, chemotherapeutic agents, antifungal 20 agents, antifungal microbial and antiviral substances, biological drugs, nutraceuticals, pesticides, chelating agents or mixtures thereof.

In accordance with the invention, the present medical device is made by a process essentially comprising the steps of:
- a) providing a support of the type considered above, for the containment and release of a drug of the abovementioned type;
- b) loading the support with a preset active substance of the abovementioned type;
- c) providing a biodegradable polymer matrix of the abovementioned type;
- d) immobilizing the support loaded with the active substance on and/or in the biodegradable polymer matrix;
- e) shaping the biodegradable polymer matrix in the form of a hollow body with a substantially circular section;
- f) cross-link the biodegradable polymer matrix;
wherein the step d), in which the support is immobilized in and/or on the biodegradable polymer matrix, may be conducted prior or subsequent to step e), wherein the biodegradable polymer matrix is shaped in the form of a hollow body with section essentially circular, and yet in which the step d), in which the support is immobilized in and/or on the biodegradable polymer matrix, coincides with the step f), in which the polymeric matrix is crosslinked.

### Figure 1

According to the above example of Figure 1 at **1** is shown a medical device according to a preferred embodiment of the present invention, with a corresponding enlarged detail, implanted in a lumen indicated with **2** of a blood vessel of which it is shown a portion, indicated with **3,** of a subject such as a human being.

In detail, the medical device **1** includes:
a hollow body indicated with **4,** having an essentially circular section and substantially made of a crosslinked biodegradable polymeric matrix, then comprising chains of monomeric units or repeating units indicated with **5,** wherein the chains of repeating units are joined in points or nodes of crosslinking;
at least one active substance associated with the hollow body **4,** which in the example of Figure 1 it is not illustrated;
and at least one support of containment and release of the at least one active substance;
in which the at least one support comprises a plurality of nanometric elements, i.e. liposomes, indicated with **6;**
and wherein the nanometric elements **6** are immobilized in and/or on biodegradable polymeric matrix covalently, through chemical bonds, at the nodes of crosslinking of the biodegradable polymer matrix.

In practice, the nanometric elements **6** are the same nodes of crosslinking of the biodegradable polymeric matrix.

In this case, advantageously, is the density of the nodes of crosslinking of the biodegradable polymeric matrix, consisting of the elements of nanometric dimensions, to modulate the release rate of the at least one active substance.

Without wishing to be bound to any specific theory, it is thought that the cross-linking density of the nodes in the biodegradable polymeric matrix can affect the ability of nanometric elements to release the at least one active substance (empty), in the sense that if the size of nanometric elements, then the nodes of cross-linking, are tightly packed between them, they will swell less and therefore empty itself less or more slowly.

The present invention is described in more detail in the following examples provided for illustrative and not limitative purpose.

### Example 1

### - Preparation of a support of nanometric dimensions: Preparation of liposomes

Liposomes are spherical cavities with a diameter of the order of 100-150 nm and a phospholipid bilayer.

The internal cavity of a liposome contains water and therefore can accommodate hydrophilic substances; the double layer, in contrast, can accommodate hydrophobic compounds.

The behavior of liposomes is studied in solutions of phosphate buffer, in particular by mixing in water a desirable ratio of sodium acid phosphate and potassium hydrogen phosphate to stabilize the physiological pH.

A desired amount of liposomes is prepared using the standard method of the thin film.

As lipid components are used soy lecithin (L-alpha-phosphatidylcholine 95%), Epikuron 200; phospholipids Epikuron 130p (33% phosphatidylcholine, 15% phosphatidylethanolamine, 16% phosphatidylinositol, phosphatidic acid 6%, purity 65%); pure cholesterol extracted from lanolin; Oleic acid 99% pure; prostacyclin analogues.

A variation of concentration of the lipid components allows to modulate their surface charge, and, therefore, their stability.

### - Loading of the support with nanometric dimensions with one or more active substances: Loading of liposomes with a drug

Lipophilic drugs have shown a high affinity for the double layer of the liposomes, and hence they result entirely encapsulated (associated) in them, while drugs consisting of compounds with partial or total hydrophilic characteristics show an efficiency of encapsulation less than 100%.

The fraction of drug (active ingredient) which is not encapsulated and that could cause side effects in a patient and also determine physical instability of the liposomes is removed.

Upload active, that is to say the standard technique of filling of the liposomes with the drug carried by an operator, is the chosen method used for medicines ionizable that allows to optimize the ratio drug/lipid.

The load, then the amount of drug encapsulated, is dependent on the concentration gradient of the drug on double layer of the liposomes, the permeability coefficient and the extra-liposomal concentration.

### - Preparation of a biodegradable polymer matrix possibly reticulated: preparation of the polymer

A biodegradable polymer matrix is prepared according to the desired polymerization techniques known in the art.

Biodegradable polymers preferred are the aliphatic polyesters polylactic acid (PLA/PLLA), poly (lactic-co-glycolic) acid (PLG).

To crosslink the biodegradable polymeric matrix cross-linking agents known in the art can be used, preferably triallyl-isocyanurate (TAIC), possibly in combination with irradiation.

For example, a content of TAIC of 3% by mass on the total mass of the polymer and a radiation dose of 50 kGy are optimum conditions for the crosslinking of PLLA, in which the density of crosslinking depends on the structure and the length of the chains of PLLA.

### - Immobilization of the nanometric support loaded with the drug on and/or in the biodegradable polymeric matrix: Immobilization of the liposomes in the polymer

Various methods can be used to immobilize the liposomes on, and/or a polymer. The methods of immobilization may include physical and chemical treatments.

### - - Physical Treatments:

- - - Adsorption (not according to the invention): adsorption of a liposome on a biodegradable polymer matrix, for example a biodegradable polymer film, is a simple method to obtain an immobilized carrier.

Basically, the liposome is attached to the biodegradable polymeric matrix by non-covalent bonds, and does not require any pre-activation of the biodegradable polymer matrix.

The interactions between the liposome and the biodegradable polymeric matrix are dependent on the surface chemistry of the biodegradable polymeric matrix and by the surface charge of the liposome.

In the immobilization by adsorption of the liposomes, in practice, the weak interactions that develop with the biodegradable polymer matrix are exploited, such as ionic or hydrophobic interactions, hydrogen bonds and van der Waals forces and, therefore, the biodegradable polymer matrix must have sufficient surface charge so that it can be a valuable substrate for the adsorption of liposomes.

For the adsorption of the liposomes on the biodegradable polymeric matrix, a liposomal suspension, and a biodegradable polymeric matrix are mixed together until a complete adsorption, after which the excess of the liposomes is removed by washing of the biodegradable polymeric matrix, in particular by passage over a column packed with Sephadex G-50.

- - - Ionic bond (not according to the invention): the immobilization of the liposomes through an ionic bond is based, primarily, on the relationship of these with a biodegradable polymer matrix containing ionic charges.

In some cases, along with the immobilization via ionic bond there can be immobilization by physical adsorption.

The main difference between the immobilization via ionic bonding and physical adsorption lies in the strength of the interaction, which is considerably greater in the first case.

The immobilization of the liposomes by ionic bond is essentially based on the same procedure used for the physical adsorption described above.

The ionic nature of the binding forces between the liposomes and the polymeric matrix is also dependent on changes in pH, temperature and charge of the support.

- - - Entrapment. The method for entrapment immobilization consists essentially in the physical caption of the liposomes in the meshes of a crosslinked biodegradable polymer matrix.

In this case liposomes loaded with the drug are mixed with a polymer (biodegradable polymer matrix), for example PLA, and then is carried out a crosslinking reaction of the polymer with formation of a crosslinked structure comprising a mesh that trap the liposomes.

The advantages of this method are the development of a high surface area available for the bond between the biodegradable polymer matrix and support of nanometric size, in a relatively small volume, and in the possibility of simultaneous immobilization of the drug and of the nanometric support.

### - - Chemical treatments

### - - - Activation plasma (not according to the invention):

Through plasma activation it is possible to functionalize the surface of a polymeric matrix with biodegradable chemical groups appropriate to be used as docking sites for the covalent immobilization of the liposomes.

In this case liposomes are prepared coated with molecules of polyethylene glycol having terminal amino groups.

The advantages of the present invention, already appeared evident in the course of the above description, can be summarized noting that it is provided a medical device in biodegradable polymeric material that allows the release of one or more active substances in a controlled manner, and hence at programmed speed and concentration, regardless of the degradation of the polymer matrix, allowing the release of an effective dose of active substance for a prolonged time interval.

Moreover, advantageously, the present medical device can also be used for only functional and not mechanical purpose, hence as a carrier for drug delivery, particularly in the management and treatment of pulmonary hypertension.

## Claims

1. Medical device comprising:
a hollow body having an essentially circular cross section substantially made of a biodegradable polymer matrix,
and at least one active substance associated with said hollow body,
**characterized in that** said medical device comprises at least one support of containment and release of said at least one active substance, wherein said at least one support comprises a plurality of nanometric-sized elements consisting of liposomes, in which said at least one support is dispersed on and/or within the biodegradable polymer matrix, in which said biodegradable polymer matrix is a polymer or a copolymer of a polylactic acid (PLA/PLLA) at least partially crosslinked and wherein said at least one support is entrapped in said polymer matrix.

2. Device according to claim 1, wherein said copolymer is a block copolymer.

3. Device according to claim 2, wherein said block copolymer is crosslinked and comprises a network mesh size of about 10 nm or less, more preferably a mesh size comprised between 6 and 10 nm, even more preferably with continuous block domains of the same monomeric unit of length comprised between 6 nm and 18 nm, even more preferably between 8 nm and 15 nm.

4. Device according to any one of the preceding claims, wherein said at least one support is dispersed on and/or within the biodegradable polymer matrix at least at an inner surface or at an inner portion of said hollow body.

5. Device according to any one of the preceding claims, wherein said at least one active substance is selected from the group comprising: cholinergic receptor stimulating agents and cholinesterase inhibitors, cholinergic receptor blocking agents, sympathomimetic agents, adrenergic receptor blockers, antihypertensive substances, venous and/or arterial vasodilator substances, substances active in the treatment of angina pectoris, substances active in the treatment of cardiac arrhythmias, diuretics, histaminic substances, serotonin, vasoactive 5 peptides, eicosanoids including prostaglandins, thromboxanes, leukotrienes and related substances for treatment of obstructive pulmonary disease, sedatives/hypnotic agents, anticonvulsant substances, substances for the treatment of Parkinson's disease, lithium based antidepressant and/or antipsychotic substances, local or general anesthetic and/or analgesic substances, substances for smooth muscle relaxation, hematopoietic growth factors, substances for the treatment of coagulation disorders, substances used for the treatment of dyslipidaemia, NSAIDs, anti-rheumatic substances, non-opioid analgesics, substances for the treatment of bones and for the treatment of mineral homeostasis, hormones, antithyroideal antagonistic substances, antidiabetics, antibiotic substances, chemotherapeutic agents, antifungal agents, antifungal microbial and antiviral substances, biological drugs, nutraceuticals, pesticides, chelating agents or mixtures thereof.

6. Process of production of a biodegradable polymer medical device according to any one of the preceding claims, comprising the steps of:
- a) providing at least one support for the containment and the release of at least one predetermined active substance, wherein said at least one support comprises a plurality of nanometric-sized elements consisting of liposomes;
- b) loading said at least one support with said at least one predetermined active substance;
- c) providing a biodegradable polymer matrix made of polylactic acid (PLA/PLLA);
- d) immobilizing said at least one support loaded with said at least one active substance on and/or within said biodegradable polymer matrix;
- e) shaping said biodegradable polymer matrix in the form of a hollow body having an essentially circular cross section;
- f) crosslinking said biodegradable polymer matrix,
wherein at least part of said step d), wherein said at least one support is immobilized within and/or on said biodegradable polymer matrix, coincides with said step f), wherein said polymer matrix is crosslinked .

7. Process according to claim 6, wherein said step d), wherein said at least one support is immobilized within and/or on said biodegradable polymer matrix, is carried out after said step e), wherein said biodegradable polymer matrix is shaped in the form of a hollow body having a substantially circular cross section.

## Patentansprüche

1. Medizinprodukt, umfassend:
einen Hohlkörper der einen im Wesentlichen kreisförmigen Querschnitt aufweist und im Wesentlichen aus einer biologisch abbaubaren Polymermatrix besteht,
und mindestens einen dem Hohlkörper zugeordneten Wirkstoff,
**dadurch gekennzeichnet, dass** das Medizinprodukt mindestens einen Träger zur Aufnahme und Freisetzung des mindestens einen Wirkstoffs umfasst, wobei der mindestens eine Träger eine Vielzahl von aus Liposomen bestehenden Elementen von nanometrischer Größe umfasst, wobei der mindestens eine Träger auf und/oder innerhalb der biologisch abbaubaren Polymermatrix dispergiert ist, wobei die biologisch abbaubare Polymermatrix ein Polymer oder ein Copolymer aus einer zumindest teilweise vernetzten Polymilchsäure (PLA/PLLA) ist und wobei der mindestens eine Träger in der Polymermatrix eingeschlossen ist.

2. Vorrichtung nach Anspruch 1, wobei das Copolymer ein Blockcopolymer ist.

3. Vorrichtung nach Anspruch 2, wobei das Blockcopolymer vernetzt ist und eine Netzmaschengrösse von etwa 10 nm oder weniger, bevorzugt eine Maschengrösse zwischen 6 und 10 nm, noch bevorzugter mit kontinuierlichen Blockdomänen derselben Monomereinheit mit einer Länge zwischen 6 nm und 18 nm, noch bevorzugter zwischen 8 nm und 15 nm, umfasst.

4. Vorrichtung nach einem der vorhergehenden Ansprüche, wobei der mindestens eine Träger auf und/oder innerhalb der biologisch abbaubaren Polymermatrix zumindest an einer Innenfläche oder an einem Innenabschnitt des Hohlkörpers dispergiert ist.

5. Vorrichtung nach einem der vorhergehenden Ansprüche, wobei der mindestens eine Wirkstoff ausgewählt ist aus der Gruppe umfassend: Stimulierungsmittel für Acetylcholinrezeptoren und Cholinesterase-Hemmer, Acetylcholinrezeptoren-Blocker, Sympathomimetika, Adrenozeptorenblocker, Antihypertensiva, venösen und/oder arteriellen Vasodilatantia, Substanzen, die bei der Behandlung von Angina pectoris aktiv sind, Substanzen, die bei der Behandlung von Herzarrhythmien aktiv sind, Diuretika, Histaminische Substanzen, Serotonin, Vasoaktive Peptide, Eicosanoide einschliesslich Prostaglandine, Thromboxane, Leukotriene und verwandte Substanzen zur Behandlung von obstruktiver Lungenerkrankung, Sedativa/Hypnotika, Antikonvulsiva, Substanzen zur Behandlung der Parkinson-Krankheit, Antidepressiva und/oder Antipsychotika auf Lithium-Basis, lokale oder allgemeine anästhetische und/oder analgetische Substanzen, Substanzen zur Glattmuskelrelaxation, hämatopoetische Wachstumsfaktoren, Substanzen zur Behandlung von Koagulationsstörungen, Substanzen zur Behandlung von Dyslipidämie, NSAIDs, Anti-Rheumatika, nicht-opioiden Analgetika, Substanzen zur Behandlung von Knochen und zur Behandlung von mineralischer Homöostase, Hormone, thyreostatische antagonistische Substanzen, Antidiabetika, Antibiotika, Chemotherapeutika, Antimykotika, antifungale mikrobielle und antivirale Substanzen, biologische Arzneimittel, Nutrazeutika, Pestizide, Chelatbildner oder Mischungen davon.

6. Verfahren zur Herstellung eines Medizinproduktes aus biologisch abbaubarem Polymer nach einem der vorhergehenden Ansprüche, umfassend die Schritte:
- a) Bereitstellen mindestens eines Trägers zur Aufnahme und Freisetzung mindestens eines vorbestimmten Wirkstoffes, wobei der mindestens eine Träger eine Vielzahl von aus Liposomen bestehenden Elementen von nanometrischer Größe umfasst;
- b) Beladen des mindestens einen Trägers mit dem mindestens einen vorbestimmten Wirkstoff;
- c) Bereitstellen einer biologisch abbaubaren Polymermatrix aus Polymilchsäure (PLA/PLLA);
- d) Immobilisieren des mindestens einen mit dem mindestens einen Wirkstoff beladenen Trägers auf und/oder innerhalb der biologisch abbaubaren Polymermatrix;
- e) Formen der biologisch abbaubaren Polymermatrix in Form eines Hohlkörpers mit im wesentlichen kreisförmigem Querschnitt;
- f) Vernetzen der biologisch abbaubaren Polymermatrix,
wobei zumindest ein Teil des Schrittes d), in dem der mindestens eine Träger innerhalb und/oder auf der biologisch abbaubaren Polymermatrix immobilisiert ist, mit dem Schritt f) zusammenfällt, wobei die Polymermatrix vernetzt ist.

7. Verfahren nach Anspruch 6, wobei der Schritt d), in dem der mindestens eine Träger innerhalb und/oder auf der biologisch abbaubaren Polymermatrix immobilisiert ist, nach dem Schritt e) durchgeführt wird, wobei die biologisch abbaubare Polymermatrix in Form eines Hohlkörpers mit im Wesentlichen kreisförmigem Querschnitt geformt ist.

## Revendications

1. Dispositif médical comprenant:
un corps creux ayant une section transversale sensiblement circulaire, essentiellement fait d'une matrice polymère biodégradable,
et au moins une substance active associée audit corps creux,
**caractérisé en ce que** ledit dispositif médical comprend au moins un support de confinement et de libération de ladite au moins une substance active, dans lequel ledit au moins un support comprend une pluralité d'éléments de taille nanométrique constitués de liposomes, dans lequel ledit au moins un support est dispersé sur et/ou dans la matrice polymère biodégradable, dans lequel ladite matrice polymère biodégradable est un polymère ou un copolymère d'un acide polylactique (PLA/PLLA) au moins partiellement réticulé et dans lequel ledit au moins un support est piégé dans ladite matrice polymère.

2. Dispositif selon la revendication 1, dans lequel ledit copolymère est un copolymère à blocs.

3. Dispositif selon la revendication 2, dans lequel ledit copolymère à blocs est réticulé et comprend une taille de maille de réseau d'environ 10 nm ou moins, plus préférablement une taille de maille comprise entre 6 et 10 nm, encore plus préférablement avec des domaines de blocs continus de la même unité monomère de longueur comprise entre 6 nm et 18 nm, encore plus préférablement entre 8 nm et 15 nm.

4. Dispositif selon l'une quelconque des revendications précédentes, dans lequel ledit au moins un support est dispersé sur et/ou dans la matrice polymère biodégradable au moins sur une surface interne ou sur une partie interne dudit corps creux.

5. Dispositif selon l'une quelconque des revendications précédentes, dans lequel ladite au moins une substance active est choisie dans le groupe comprenant: agents stimulant les récepteurs cholinergiques et inhibiteurs de la cholinestérase, agents bloquant les récepteurs cholinergiques, agents sympathomimétiques, bloquants des récepteurs adrénergiques, substances antihypertensives, substances vasodilatatrices veineuses et/ou artérielles, substances actives dans le traitement de l'angine de poitrine, substances actives dans le traitement des arythmies cardiaques, diurétiques, substances histaminiques, sérotonine, peptides 5 vasoactifs, eicosanoïdes y compris prostaglandines, thromboxanes, leucotriènes et substances apparentées pour le traitement des maladies pulmonaires obstructives, agents sédatifs/hypnotiques, substances anticonvulsives, substances pour le traitement de la maladie de Parkinson, substances antidépressives et/ou antipsychotiques à base de lithium, substances anesthésiques et/ou analgésiques locales ou générales, substances pour la relaxation des muscles lisses, facteurs de croissance hématopoïétiques, substances pour le traitement des troubles de la coagulation, substances utilisées pour le traitement de la dyslipidémie, AINS, substances anti-rhumatismales, analgésiques non opiacés, substances pour le traitement des os et pour le traitement de l'homéostasie minérale, hormones, substances antagonistes antithyroïdiennes, antidiabétiques, substances antibiotiques, agents chimiothérapeutiques, agents antimycosiques, substances microbiennes et antivirales antimycosiques, médicaments biologiques, nutraceutiques, pesticides, agents de chélation ou leurs mélanges.

6. Procédé de production d'un dispositif médical en polymère biodégradable selon l'une quelconque des revendications précédentes, comprenant les étapes de:
- a) fournir au moins un support pour le confinement et la libération d'au moins une substance active prédéterminée, dans lequel ledit au moins un support comprend une pluralité d'éléments de taille nanométrique constitués de liposomes;
- b) charger ledit au moins un support avec ladite au moins une substance active prédéterminée;
- c) fournir une matrice polymère biodégradable faite d'acide polylactique (PLA/PLLA);
- d) immobiliser ledit au moins un support chargé avec ladite au moins une substance active sur et/ou dans ladite matrice polymère biodégradable;
- e) modeler ladite matrice polymère biodégradable sous la forme d'un corps creux ayant une section transversale essentiellement circulaire;
- f) réticuler ladite matrice polymère biodégradable,
dans lequel au moins une partie de ladite étape d), dans laquelle ledit au moins un support est immobilisé dans et/ou sur ladite matrice polymère biodégradable, coïncide avec ladite étape f), dans laquelle ladite matrice polymère est réticulée.

7. Procédé selon la revendication 6, dans lequel ladite étape d), dans laquelle ledit au moins un support est immobilisé dans et/ou sur ladite matrice polymère biodégradable, est réalisée après ladite étape e), dans laquelle ladite matrice polymère biodégradable est modelée sous la forme d'un corps creux ayant une section transversale sensiblement circulaire.
